# EUROPÄISCHE PATENTSCHRIFT

(11) **EP 0 754 013 B1**
(45) Veröffentlichungstag und Bekanntmachung des Hinweises auf die Patenterteilung: **04.11.1998**
(21) Anmeldenummer: 95913000.6
(22) Anmeldetag: 04.04.1995
(51) Int. Cl.: A61B 17/128

(54) **VORRICHTUNG ZUM SETZEN VON HAUTKLAMMERN**
DEVICE FOR POSITIONING SKIN CLIPS
DISPOSITIF POUR LE POSITIONNEMENT D'AGRAFES CHIRURGICALES

(30) Priorität: 08.04.1994 CH 1053/94
(43) Veröffentlichungstag der Anmeldung: 22.01.1997
(73) Patentinhaber: WALDER-UTZ, Alice, Dr., CH-8006 Zürich (CH)
(72) Erfinder: WALDER-UTZ, Alice, CH-8006 Zürich (CH); DUBACH, Werner, Fritz, CH-8124 Maur (CH)
(74) Vertreter: Patentanwaltsbüro Feldmann AG
(86) Internationale Anmeldenummer: CH9500075
(87) Internationale Veröffentlichungsnummer: WO9527442

(56) Entgegenhaltungen:
- EP-A- 0 090 484
- EP-A- 0 469 524
- WO-A-83/02887
- WO-A-88/01487
- WO-A-93/09721
- US-A- 4 671 278

## Beschreibung

Die vorliegende Erfindung betrifft eine Vorrichtung zum Setzen von Hautklammern aus einem in deren Gehäuse untergebrachten Magazin.

Aus der US-A-3'601'127 und der FR-A-419'096 sind äusserst einfache, federelastisch die Wundränder zusammenklemmende Hautklammern bekannt. Die erstgenannte Klammer besteht aus zwei Metallstegen, mit etwa U-förmigem Querschnitt, die über einen gummielastischen Streifen miteinander verbunden sind. Die Wundränder werden durch krallenartige Spitzen gehalten. Hierbei werden die Wundränder perforiert und damit verbundene Infektionen und traumatische Reaktionen sind nicht auszuschliessen. Die zweitgenannten Wundklammern bestehen aus einem einzigen gestanzten und gebogenen Teil aus rostfreiem Federstahl, bei dem die Wundränder zusammengequetscht und aufgestülpt werden und Rückhaltekanten ein Herausgleiten der Wundränder vermeiden sollen. Medizinisch ist auch diese Lösung nicht optimal, da auch hier die Wundränder im aufgestülpten Bereich perforiert werden können, wobei diese Insektionen einer optischen Kontrolle nicht zugänglich sind. Eventuell auftretende Infektionsherde werden somit relativ erst spät entdeckt.

Einen ähnlichen Typ einer Wundklammer ist ferner aus der US-A-4'217'902 bekannt. Diese Wundklammer weist Mittel auf, mittels derer sie mit einer Zange ergriffen und gesetzt werden kann. Die vorher beschriebenen Wundklammern werden üblicherweise von Hand durch den Chirurgen gesetzt. Diesbezüglich am komfortabelsten ist eine von der Anmelderin entwickelte Hautklammer gemäss der EP-B-224'500. Hier werden die Wundränder zwischen zwei gewellten Andruckkanten aufeinander gepresst. Hierbei tritt keine Perforation ein. Das bleibende Narbenbild ist folglich frei von Einstichstellen, wie sie bei den genähten Wundverschlüssen oder entlang von Wundrändern, die mittels der vorgenannten Wundklammern oder mittels den bekannten Heftklammern zusammengehalten wurden, entstehen..

Trotzdem die genannten Heftklammern relativ tief beidseits der Wunde in die Haut eingepresst werden und folglich traumatische Zustände im Wundbereich häufig feststellbar sind und auch die Gefahr der Infektion relativ gross ist, haben sich diese Heftklammersysteme stark verbreitet. Dies liegt daran, dass sie besonders anwenderfreundlich sind. Sie werden in Einwegvorrichtungen geliefert, die aus einem Gehäuse bestehen, in welchem ein Magazin mit einer fest vorgegebenen Anzahl Hautklammern eingebracht ist. Sind die heftklammerförmigen Hautklammern gesetzt und folglich das Magazin leer, oder der Wundverschluss vollständig gemacht, so wird die gesamte Vorrichtung inklusive eventuell nicht gebrauchter Hautklammern weggeworfen.

Aus der WO-A-83-2887 ist eine Vorrichtung bekannt, bei der chirurgische Klammern zwischen einem stabförmigen Förderer und einer Leiste nur einseitig in einem Gehäuse geführt sind. Ferner wird die unterste Klammer am distalen Ende des Instrumentes hier mittels Rampen beim Ausdrücken nicht gespreizt, sondern zusammengedrückt.

Ferner ist aus der EP-A-090'484 eine Instrument bekannt, mit einschiebbarem Magazin, in dem die chirurgischen Klammern untergebracht sind. Die Klammern im Magazin werden von einem oberen Magazinteil bewegt. Die distale Klammer wird bei der eigentlichen Setz- und Spreizbewegung von einem zweiten fördernden Element bewegt.

Die vorliegende Erfindung hat sich zur Aufgabe gestellt, erstmals eine Vorrichtung zum Setzen von Hautklammern aus einem in deren Gehäuse untergebrachten Magazin zu schaffen, wobei die Hautklammern vom Typ der eingangs genannten Art sind. Dies verlangt eine gegenüber den bekannten Vorrichtungen anders gestaltete Magazinierung und Förderung der Hautklammern.

Die vorgenannte Aufgabe löst eine Vorrichtung mit den Merkmalen des Patentanspruches 1. Da die hier zum Einsatz gelangenden Hautklammern nicht lediglich die Gestalt von gebogenen Drahtabschnitten haben, wie dies bei Heftklammern der Fall ist, können diese nicht nebeneinander angeordnet werden, wie dies bei den Geräten zu Applizieren von Heftklammern der Fall ist, sondern müssen in einer gestapelten Form übereinander angeordnet werden.

Vorteilhafte Ausgestaltungsformen des Erfindungsgegenstandes sind in den abhängigen Ansprüchen beansprucht und deren Bedeutung und Wirkungsweise sind in der nachfolgenden Beschreibung erläutert.

In der anliegenden Zeichnung ist ein bevorzugtes Ausführungsbeispiel des Erfindungsgegenstandes dargestellt und in der Beschreibung mit Bezug auf die Figuren erklärt. Es zeigt:
- Figur 1 -: eine Frontansicht der erfindungsgemässen Vorrichtung mit einem auswechselbaren Magazin;
- Figur 2 -: dieselbe Vorrichtung nach Figur 1 nach der Entfernung der frontseitigen Gehäuseschale.
- Figur 3 -: zeigt den stabförmigen Förderer für sich allein dargestellt mit darauf stapelartig aufgereihten Hautklammern in grösserem Massstab;
- Figur 4 -: zeigt eine Hautklammer entsprechend jenen nach Figur 3 für sich alleine in der Aufsicht in etwas kleinerem Massstab und
- Figur 5 -: eine Variante der Hautklammer in derselben Darstellung wie nach Figur 4 und schliesslich
- Figur 6 -: eine nochmals leicht geänderte Ausführung der Hautklammer in der Seitenansicht im selben Massstab wie in Figur 3.

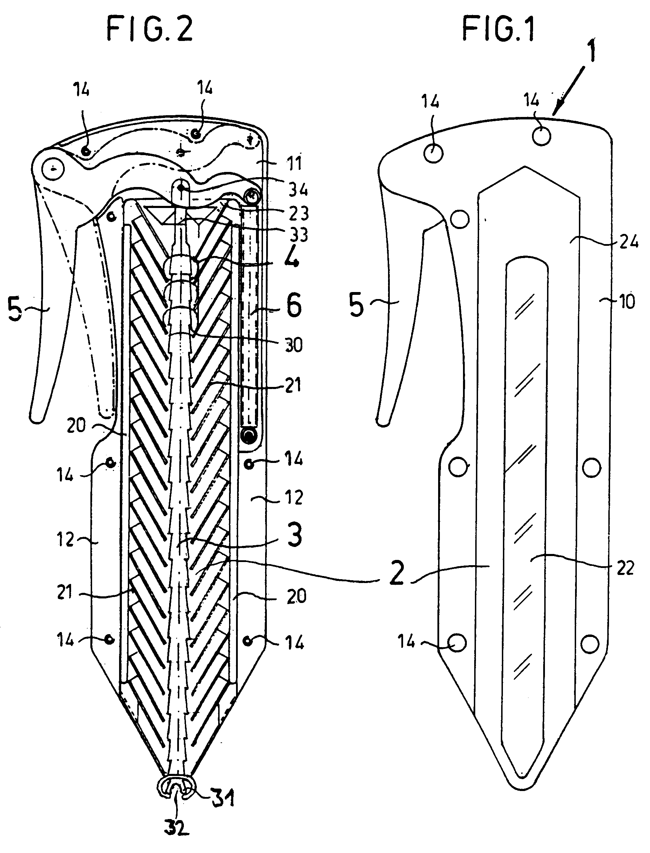

Die Figur 1 zeigt eine bevorzugte Ausführungsform der Vorrichtung zum Setzen von Hautklammern etwa in der natürlichen Grösse. Das gesamte Gehäuse ist mit der Bezugszahl 1 bezeichnet. Es besteht in der vorliegenden Ausführungsform aus zwei Gehäuseschalen, nämlich der frontseitigen Gehäuseschale 10 und der rückseitigen Gehäuseschale 11. Beide Gehäuseschalen liegen deckungsgleich übereinander und sind mittels Verbindungsstellen 14 miteinander verbunden. Diese Verbindungsstellen 14 können Schraubverbindungen, genietete oder geschweisste Verbindungen sein. In der Serienfabrikation wird man die in der Kunststofftechnik verbreitete, reine Steckverbindung wählen, mittels derer eine kombinierte Form-Kraftschlussverbindung erzeugt wird. So brauchen dann die beiden Halbschalen 10 und 11 lediglich deckungsgleich übereinander gelegt zu werden und zusammengepresst werden. Im geschlossenen Zustand der Vorrichtung erkennt man noch einen Betätigungshebel 5 sowie das Magazin 2. Das Magazin 2 hat mindestens eine frontseitige Abdeckung mit einem Sichtfenster 22, welches die Sichtkontrolle über die noch vorhandenen Hautklammern erlaubt. Prinzipiell kann selbstverständlich das gesamte Magazin 2 aus transparentem Material gefertigt sein. Vorzugsweise besteht dieses genauso wie die Hautklammern aus einem hochwertigen, für Medizinalzwecke zugelassenen Kunststoff.

Der genauere Aufbau der erfindungsgemässen Vorrichtung ist aus der Figur 2 ersichtlich, nach Entfernung der frontseitigen Gehäuseschale 10. Hier erkennt man, dass mindestens die rückseitige Gehäuseschale 11 versteifte Seitenwangen 12 aufweist, die so dick sind, dass sie zwischen sich einen Schacht freilassen, der breit genug ist, das Magazin 2 aufzunehmen. Das Magazin 2 besteht aus zwei seitlichen Leisten 20, an denen schräg nach unten zum Zentrum hin gerichtete, parallel zueinander verlaufende Rückhalteplättchen 21 angeformt sind. Die Enden zweier einander gegenüberliegender, federnder Rückhalteplättchen 21 nähern sich bis auf eine Distanz, die geringer ist als die Schulterbreite der im Magazin aufzunehmenden Hautklammern 4. Dies bewirkt, dass bei einer Abwärtsbewegung der Hautklammern 4 jeweils zwei einander gegenüberliegende Rückhalteplättchen 21 durch die Hautklammer federnd nach aussen gebogen werden, während bei einer Reaktionskraft der Hautklammern nach oben die Federplättchen als Sperren wirken, weil die Reaktionskräfte mehr oder weniger exakt in die Längsrichtung der Federplättchen in diese eingeleitet werden.

Die beiden seitlichen Leisten 20, mit den daran angeformten federnden Rückhalteplättchen 21 können direkt einen Teil der versteiften Seitenwangen 12 bilden oder aber über einer gemeinsamen Rückwand 23 ein Magazin 2 als selbständiges Bauteil formen. Im letzteren Fall wird dieses Bauteil von einer frontseitigen Abdeckplatte abgeschlossen. Ein geladenes Magazin als auswechselbares Teil der Vorrichtung besteht folglich aus den beiden seitlichen Leisten 20 mit den federnden Rückhalteplättchen 21 sowie der die beiden Leisten verbindenden Rückwand 23 und Abdeckplatte 24, sowie schliesslich einem das Magazin in Längsrichtung zentrisch durchsetzenden, stabförmigen Förderer 3. Der Förderer 3 hat die Gestalt eines sägezahnförmig ausgebildeten, schwertartigen Stabes, dessen in Abgaberichtung der Hautklammern unterstes Ende erweitert ist. In der Zeichnung sind die Sägezähne mit 30, die endständige Erweiterung mit 31 bezeichnet. Die endständige Erweiterung 31 weist selber wieder eine bogenförmige Ausnehmung 32 auf. Diese Ausnehmung dient dazu, die unterste, gespreizte Hautklammer über die seitlichen nach oben gewölbten Wundränder zu schieben. Das obere Ende des stabförmigen Förderers 3 weist einen Hals 33 mit einer Befestigungsöse 34 auf. Die Oese 34 dient der lösbaren Befestigung des stabförmigen Förderers 3 mit den innenliegenden Hebelarm des Betätigungshebels 5. Zum Auswechseln des Magazins 2 muss lediglich die Verbindung zwischen dem stabförmigen Förderer 3 und dem Betätigungshebel 5 gelöst werden, worauf das gesamte Magazin 2 ausgewechselt werden kann.

Neben der hier dargestellten, bevorzugten Ausführungsform sind weitere Ausgestaltungen des Magazines 2 durchaus denkbar. So kann es genügen, dass das Magazin 2 lediglich einseitig eine Leiste 20 mit federnden Rückhalteplättchen 21 aufweist. Eine weitere Variante besteht darin, dass jeweils abwechslungsweise je ein Federplättchen von links und dann von rechts auf zwei benachbarte, im Magazin gestapelte Hautklammern wirkt. Dies bedingt lediglich eine etwas verstärkte Form der Federplättchen 21. Am innenliegenden Hebelarm des Betätigungshebels 5 greift endseitig eine Rückholfeder 6 an. Die Rückholfeder 6 liegt dabei in einer Ausnehmung 13 in der einen, versteiften Seitenwange 12.

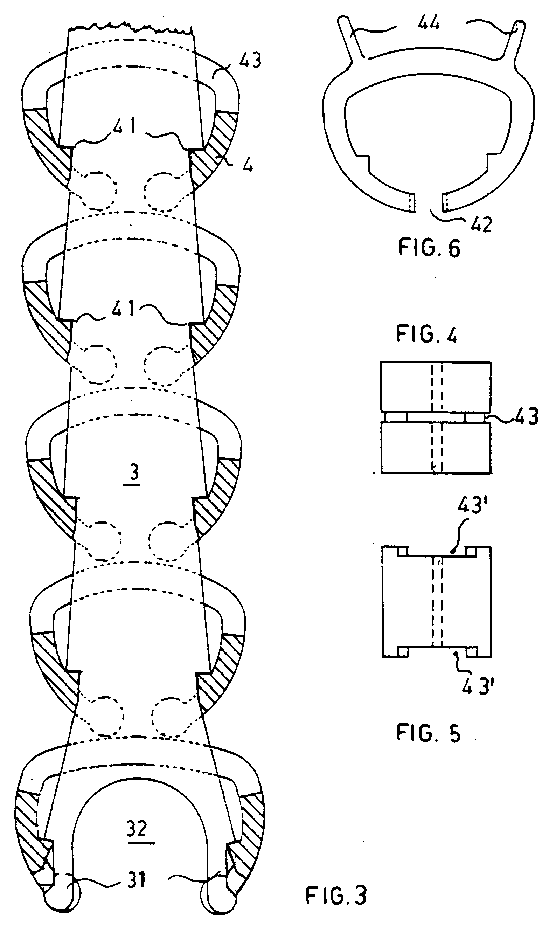

Aus den Figuren 3 - 6 gehen verschiedene mögliche Ausführungsformen der für die erfindungsgemässe Vorrichtung gebräuchlichen Hautklammern hervor. Die hier verwendeten Hautklammern haben etwa die Form einer Hülse mit einem in Längsrichtung verlaufenden Klemmspalt 42. Die Ausführungsformen der Hautklammer 4 gemäss den Figuren 3 und 4 haben zusätzlich eine zentrische, senkrecht zum Klemmspalt verlaufende zentrale Führung 43. Durch diese Führung 43 ist der stabförmige Förderer 3 hindurchgeführt. An den seitlichen Schenkeln nach innen ragende Rückhalterippen 41 wirken mit dem stabförmigen Förderer 3 zusammen. Die Sägezähne 30 des stabförmigen Förderers 3 liegen auf den Rückhalterippen 41 jeweils auf. Statt der zentralen Führung 43 kann die Hautklammer 4 auch mit seitlichen Führungen 43' versehen sein. In diesem Fall muss dann die erfindungsgemässe Vorrichtung mit zwei parallelen stabförmigen Förderern 3 ausgestaltet sein, die exakt entsprechend der Breite der Wundklammer parallel zueinander geführt sind. Diese Variante ist im Anspruch 1 berücksichtigt, indem von mindestens einem stabförmigen Förderer gesprochen wird. Prinzipiell schwellen die im Klemmspalt 42 liegenden Wundränder während der Heilung ab, so dass die Wundklammer 4 mit Leichtigkeit abgezupft werden kann. Ist jedoch die Entfernung der Wundklammer zur Wundkontrolle erwünscht, so kann dies mittels einer entsprechenden Spreizzange erfolgen oder die Wundklammer 4 kann wie das Beispiel in Figur 6 zeigt mit zwei seitlichen nach aussen und oben gerichteten Halterippen 44 versehen sein. Diese lassen sich so gestalten, dass dadurch die Stapelung der Hautklammern auf dem stabförmigen Förderer 3 nicht beeinflusst wird.

Zur Betätigung der erfindungsgemässen Vorrichtung drückt der Benützer auf den Betätigungshebel 5. Dabei wird der im Gehäuse 1 liegende Arm des Betätigungshebels nach oben bewegt und zieht dabei den stabförmigen Förderer 3 mit nach oben. Die auf dem Förderer 3 aufgereihten Hautklammern 4 werden durch die Federplättchen 21 daran gehindert, diese Bewegung mitzumachen und verbleiben somit auf dem selben Niveau, während der Förderer sich nach oben bewegt. Aus der Figur 3 erkennt man, dass folglich die Klammern von den Sägezähnen 30 entfernt werden und die Klammern leicht gespreizt werden, und zwar soweit, bis sie über den nächsten Sägezahn 30 des Förderers 3 hinweggeglitten sind. Nun lässt man den Betätigungshebel 5 los, worauf die Rückholfeder 6 den Hebelarm des Betätigungshebels 5 nach unten zieht und folglich auch den stabförmigen Förderer 3 nach unten bewegt. Da nun die Hautklammern wiederum mit ihren jeweiligen Rückhalterippen 41 hinter den entsprechenden Sägezähnen 30 des Förderers 3 anliegen, werden sie mit nach unten bewegt, wobei gleichzeitig die federnden Rückhalteplättchen 21 nach aussen gebogen werden. Sämtliche Wundklammern sind nun auf dem stabförmigen Förderer eine Position tiefer angeordnet als zuvor, mit Ausnahme der damals bereits zuunterst angeordneten Hautklammer. Diese befindet sich auf dem erweiterten Teil 31 und ist soweit gespreizt, dass die Wundränder der zu verschliessenden Wunde mit Leichtigkeit in den gespreizten Klemmspalt 42 eingeführt werden können, der in dieser Lage etwa der Breite der bogenförmigen Ausnehmung 32 des Förderers 3 entspricht. Bei der nächsten Betätigung wird diese unterste Klammer vom Förderer abgeschoben und schnappt federnd zu.

Da die erfindungsgemässe Vorrichtung mindestens bezüglich dem Gehäuse und der Betätigungsvorrichtung nicht mehr als Einwegvorrichtung benutzt wird, wird man diese Teile aus besonders widerstandsfähigem Material fertigen, welches sich mehrfach sterilisieren lässt. So können diese Teile beispielsweise auch aus einem Aluminiumspritzguss gefertigt sein. Aber auch hochwertige Kunststoffe kommen durchaus in Frage.

## Patentansprüche

1. Vorrichtung zum Setzen von Hautklammern (4) aus einem in deren Gehäuse (1) untergebrachten Magazin (2), wobei die Vorrichtung mindestens einen auf sämtliche im Magazin (2) gehaltenen Hautklammern (4) wirkenden stabförmigen Förderer (3) aufweist, mittels dem alle Hautklammern (4) gleichzeitig gefördert und die in der distalen Position befindliche Hautklammer in eine gespreizte Lage bringbar ist, wobei der Förderer (3) die Gestalt eines beidseitig sägezahnförmig (30) ausgebildeten schwertartigen Stabes hat, dessen in Abgaberichtung der Hautklammern distales Ende erweitert (31) ist, und dass das Magazin (2) beidseitig je eine Leiste (20) mit federnden Rückhalteplättchen (21) aufweist, die somit an alle im Magazin vorhandenen Hautklammern (4) beidseitig anliegen und deren Bewegung nur in einer Richtung zulassen.

2. Vorrichtung nach Anspruch 1, dadurch gekennzeichnet, dass das Magazin (2) ein Teil des Gehäuses (1) der Vorrichtung ist.

3. Vorrichtung nach Anspruch 1, dadurch gekennzeichnet, dass das Magazin (2) ein auswechselbarer, in das Gehäuse (1) einsetzbarer Teil ist.

4. Vorrichtung nach Anspruch 1, dadurch gekennzeichnet, dass die Vorrichtung ein Betätigungsmittel (5) aufweist, mittels dem der stabförmige Förderer (3) mindestens annähernd um die Distanz der Länge eines Sägezahnes in eine annähernd translatorische Bewegung repetitiv bewegbar ist.

5. Vorrichtung nach Anspruch 4, dadurch gekennzeichnet, dass das Betätigungsmittel ein Hebel (5) ist, der mittels einer Feder (6) jeweils in seine Ausgangsposition zurückbringbar ist.

6. Vorrichtung nach Anspruch 4, dadurch gekennzeichnet, dass der stabförmige Förderer (3) lösbar (34) mit dem Betätigungsmittel (5) verbindbar ist.

7. Vorrichtung nach Anspruch 3, dadurch gekennzeichnet, dass das im Gehäuse (1) einsetzbare, auswechselbare Magazin (2) den stabförmigen Förderer (3) mitumfasst.

8. Vorrichtung nach Anspruch 3, dadurch gekennzeichnet, dass das als auswechselbares, in das Gehäuse (1) einsetzbare Teil gestaltete Magazin (2) die Form einer Kunststoffhülse aufweist und mindestens teilweise (22) transparent gestaltet ist.

## Claims

1. Device for applying skin clips (4) comprising a magazine (2) contained in a housing (1), the device having at least one rod-like feeder (3) acting on all the skin clips (4) contained in the magazine (2), by means of which feeder all the skin clips (4) are fed simultaneously, the skin clip in the distal position being capable of being brought into a splayed position and the feeder (3) having the form of a sword-like rod with a saw-tooth profile (30) on each side, with its distal end (31) widened in the feed direction, and in that the magazine (2) has on each side a rail (20) with sprung ratchet plates (21) which thus rest against each side of all the skin clips (4) contained in the magazine and permit them to move in only one direction.

2. Device according to Claim 1, characterised in that the magazine (2) forms part of the housing (1) of the device.

3. Device according to Claim 1, characterised in that the magazine (2) is an exchangeable part which can be inserted in the housing (1).

4. Device according to Claim 1, characterised in that the device has an actuating means (5) by means of which the rod-like feeder (3) is repetitively movable by at least approximately the distance of the length of one saw tooth in an approximately translatory movement.

5. Device according to Claim 4, characterised in that the actuating means is a lever (5) which can be returned to its starting position after each movement by means of a spring (6).

6. Device according to Claim 4, characterised in that the rod-like feeder (3) is connected detachably (34) to the actuating means (5).

7. Device according to Claim 3, characterised in that the exchangeable magazine (2) which can be inserted in the housing (1) also includes the rod-like feeder (3).

8. Device according to Claim 3, characterised in that the magazine (2) formed as an exchangeable part insertable in the housing (1) has the form of a plastic sheath and is at least partly (22) transparent.

## Revendications

1. Dispositif pour le positionnement d'agrafes chirurgicales (4) constitué d'un magasin (2) placé dans le boîtier (1) du dispositif, ce dispositif présentant au moins un transporteur (3) en forme de tige agissant sur toutes les agrafes chirurgicales (4) retenues dans le magasin (2), au moyen duquel transporteur toutes les agrafes chirurgicales (4) sont transportées simultanément et au moyen duquel l'agrafe chirurgicale se trouvant en position distale peut être amenée en position écartée, le transporteur (3) ayant la forme d'une tige similaire à une lame formée en dents de scie (30) des deux côtés, dont l'extrémité distale dans le sens de distribution des agrafes chirurgicales est élargie (31), et le magasin (2) présentant respectivement, des deux côtés, une barrette (20) avec des plaquettes de retenue (21) à ressort qui sont ainsi adjacentes des deux côtés à toutes les agrafes chirurgicales (4) se trouvant dans le magasin et qui permettent le mouvement des agrafes dans un seul sens.

2. Dispositif selon la revendication 1, **caractérisé en ce que** le magasin (2) est une pièce du boîtier (1) du dispositif.

3. Dispositif selon la revendication 1, **caractérisé en ce que** le magasin (2) est une pièce interchangeable, insérable dans le boîtier (1).

4. Dispositif selon la revendication 1, **caractérisé en ce que** le dispositif présente un organe d'actionnement (5) au moyen duquel le transporteur (3) en forme de tige est déplaçable par répétitions au moins d'environ la distance égale à la longueur d'une dent de scie dans un mouvement à peu près de translation.

5. Dispositif selon la revendication 4, **caractérisé en ce que** l'organe d'actionnement est un levier (5) qui peut être ramené dans sa position initiale respective au moyen d'un ressort (6).

6. Dispositif selon la revendication 4, **caractérisé en ce que** le transporteur (3) en forme de tige est raccordé à l'organe d'actionnement (5) de manière amovible (34).

7. Dispositif selon la revendication 3, **caractérisé en ce que** le magasin (2) interchangeable, insérable dans le boîtier (1) contient le transporteur (3) en forme de tige.

8. Dispositif selon la revendication 3, **caractérisé en ce que** le magasin (2) conçu en forme de pièce interchangeable, insérable dans le boîtier (1) présente la forme d'une douille plastique et est formé au moins en partie (22) de manière transparente.
